# EUROPEAN PATENT SPECIFICATION

(11) **EP 4 289 922 B1**
(45) Date of publication and mention of the grant of the patent: **23.07.2025**
(21) Application number: 22177794.9
(22) Date of filing: 08.06.2022
(51) Int. Cl.: C12M 1/00, C12M 1/34

(54) **VERTICAL THERMAL GRADIENT EQUIPMENT AND METHOD FOR PROGRESSIVE SPERM SORTING**
VERTIKALE THERMISCHE GRADIENTENAUSRÜSTUNG UND VERFAHREN ZUR PROGRESSIVEN SPERMASORTIERUNG
ÉQUIPEMENT ET PROCÉDÉ À GRADIENT THERMIQUE VERTICAL POUR LE TRI PROGRESSIF DE SPERME

(43) Date of publication of application: 13.12.2023
(73) Proprietor: iPreg Incorporation, Taipei City 106429 (TW)
(72) Inventor: CHANG, Ching-Wen, 106429 Taipei City (TW); CHUNG, Chen-Yen, 106429 Taipei City (TW); HUANG, Chung-Hsien, 106429 Taipei City (TW); LI, Bor-Ran, 106429 Taipei City (TW)
(74) Representative: Viering, Jentschura & Partner mbB Patent- und Rechtsanwälte

(56) References cited:
- WO-A1-2020/148474
- KR-B1- 101 088 984
- LEUNG ERICA T Y ET AL: "Simulating nature in sperm selection for assisted reproduction", NATURE REVIEWS. UROLOGY, NATURE PUBL. GROUP, US, vol. 19, no. 1, 5 November 2021 (2021-11-05), pages 16 - 36, XP037659875, ISSN: 1759-4812, [retrieved on 20211105], DOI: 10.1038/S41585-021-00530-9

## Description

### BACKGROUND OF THE INVENTION

### Field of the Invention

This invention relates generally to apparatus and methods for sperm collection, and more particularly to apparatus and methods for high yielding sperm populations that are enriched with health and DNA intact sperm cells.

### Background

Infertility is a major health problem worldwide and is estimated to affect 8-12% of couples in the reproductive age group (Agarwal et. al., Lancet 2021; 10271:319-333). Assisted reproductive technology (ART) such as in-vitro fertilization (IVF) and intracytoplasmic sperm injection (ICSI) has been widely used for infertility treatment.

While selecting highly motile and morphologically normal sperm is the obvious key to *in vitro* fertilization (IVF) and intracytoplasmic sperm injection (ICSI) success. The performance of current standard ART procedures, such as swim-up technique and density gradient separation, is not entirely satisfactory due to ROS generation and increasing the level of oxidative DNA damage in sperm cells (Punjabi et al., J Assist Reprod Genet. 2019; 36:1413-21).

Horizontal microfluidic chips enable the use of small volumes for the separation of sperm into viable and motile sperm from non-viable or non-motile sperm. Only sperms with high motility could reach the contracted region of the channel against the flow field and be transported to the outlet. Such devices can be found, for example, in patent publications US 10532357, CN 102242055B, and US 10450545. However, the process is often a time-consuming task and can have severe volume restrictions.

The device disclosed in patent publication US 10,422,737 incorporates a filter to cause sperm to move against the filter and gravity to reach the outlet. However, when patients with low sperm concentration and/or low motility, the separation result of the low yield and lower quality after sorting need to be overcome. Especially, men's sperm has been decreased in number and getting worse in swimming for some time now.

Sperm thermotaxis is one of the mechanisms for selecting capacitated spermatozoa to fertilize the oocyte. Thermotaxis is dependent on a temperature gradient established within the fallopian tube. Guided by this gradient, capacitated mammalian spermatozoa can swim away from the utero-tubal junction towards the warmer temperature where the oocyte awaits. In a recent publication, spermatozoa selection by thermotaxis in mice and human possess higher DNA integrity and increased blastocyst production as well as live birth rate of ICIS (Pérez-Cerezales et al. Scientific Reports 2018, 8:2902.). In addition, a mild heat treatment of asthenozoospermic males increased the number of motile sperm and pregnancy rate ( Küçük et al. J Assist Reprod Genet 2008, 25:235-238).

Current methods of sperm selection by thermotaxis were operating on a petri dish or microfluidic chip with a horizontal thermal gradient provided by a laboratory hot plate or resistive heater (Pérez-Cerezales et. al., Scientific Reports 2018; 8:2902). Li et. al. disclosed a horizontal temperature-gradient system for spermatozoa isolation (CN 108504563A). Leung et al. propose a conceptual sperm selection model using a microfluidic system with multiple selection mechanisms, including thermal and chemoattractant horizontal gradients (Leung et al., NATURE REVIEWS 2021, vol. 19, no. 1, pages 16-36). However, using the methods described above did not provide the throughput needed to meet IVF criteria. Furthermore, in a horizontal temperature gradient system, to establish an effective temperature difference, the hot and cold reservoirs need to be separated by a distance, resulting in space constraints for the system. Besides, there's no thermotaxis-based device to assist the sorting of motile spermatozoa on the market so far.

### SUMMARY OF INVENTION

Therefore, to increase the quantity and quality of spermatozoa selection and minimize a poor selection made by the operator and to standardize spermatozoa selection, the present invention provides a vertical temperature-gradient sperm sorting device for spermatozoa isolation.

The present invention relates to an apparatus according to claim 1 for processing sperm, comprising: an upper chamber and a bottom chamber arranged vertically; a collection port connected to the upper chamber; an injection port connected to the bottom chamber; a porous layer placed at the interface between the upper and bottom chamber to allow particles communication between them and delay heat exchange therebetween; and a temperature control unit means for maintaining the temperature of upper chamber higher than that of the bottom chamber, wherein the temperature control unit comprises a heating source placed on top of the upper chamber to heat the upper chamber by contacting.

In some embodiments, the porous layer is a biocompatible semipermeable membrane.

In some preferred embodiments, the biocompatible semipermeable membrane is a polycarbonate or polyvinyl alcohol track-etched membrane.

In some embodiments, the pore diameter of the porous layer is between 8 to 20 µm.

In some embodiments, the upper chamber comprises an inclined ceiling, the lower end of the inclined ceiling is close to the collection port and the higher end of the inclined ceiling is away from the collection port.

In some preferred embodiments, the inclined ceiling comprises a strip vent at the higher end to release air bubbles.

In some embodiments, the inclined ceiling comprises a plurality of through-holes to ventilate the upper chamber.

The present invention also relates to a method according to claim 8 for processing sperm using the said apparatus, comprising: providing the said apparatus; injecting a population of unsorted sperm into the bottom chamber through the injection port; injecting a compatible buffer into the upper chamber through the collection port; maintaining the temperature of the upper chamber higher than that of the bottom chamber; incubating a period; harvesting a population of sorted sperm from the upper chamber through the collection port.

The temperature control unit comprises a heating source placed on top of the upper chamber in thermal contact with the upper chamber.

In some embodiments, the temperature of the upper chamber is higher than that of the bottom by 1 to 4 °C.

In some preferred embodiments, the temperatures of the upper and bottom chambers are maintained between 35 to 38 °C and 30 to 36 °C, respectively.

In some embodiments, the period is between 15 to 30 minutes.

In some embodiments, the compatible buffer is a sperm washing medium (bicarbonate or HEPES buffered).

### BRIEF DESCRIPTION OF THE DRAWINGS

Fig. 1 is the schematic illustration of the device design and working principle. There is one injection port **(11)** for injecting the raw semen sample and one collection port **(21)** for the buffer injection and collection of purified spermatozoa. The upper chamber **(20)** and bottom chamber **(10)** are separated by a porous layer **(30).** A temperature control unit **(400)** was mounted to generate a vertical temperature gradient, making the temperature of the upper chamber **(20)** higher than that of the bottom chamber **(10).**

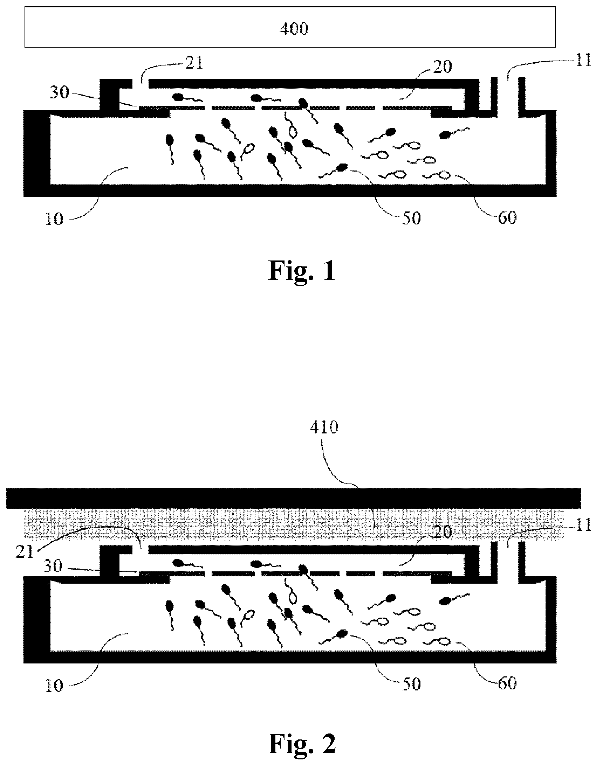

Fig. 2 is the schematic illustration of the device design and working principle. There is one injection port **(11)** for injecting the raw semen sample and one collection port **(21)** for the buffer injection and collection of sorted spermatozoa. The upper chamber **(20)** and the bottom chamber **(10)** are separated by a porous layer **(30).** To generate a vertical temperature gradient, the temperature control unit **(400)** can be a heating source **(410)** such as a mild heating pad or a hand-warming device. The heating source **(410)** heats the buffer in the upper chamber **(20)** by contacting (e.g., being placed on) the upper chamber **(20).**

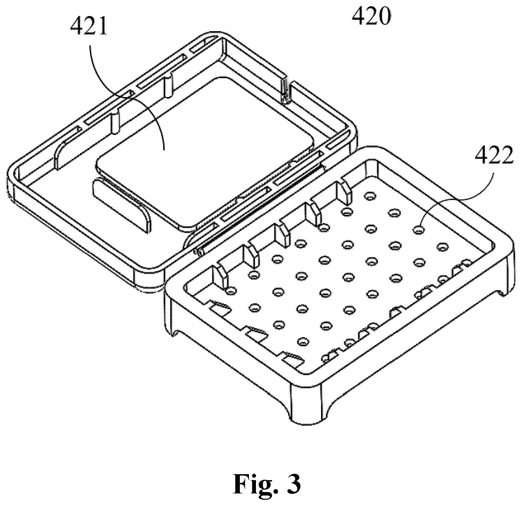

Fig. 3 depicts an example of a vertical temperature gradient device.
Fig. 4 shows the temperature difference between the upper chamber (20) and bottom chamber **(10)** in the presence or absence a porous layer (30) when providing the heat source **(410)** on the top of the device, in the device with a membrane the ΔT is approximately 4 °C; but in the device without a membrane, the ΔT is approximately 0.8 °C.
Figs. 5 shows spermatozoa sorting with or without a temperature (tm) gradient system. (A) The concentration of sperms (/mL) after sorting. (B) The percentage of progressive sperm (PR) recovery rate (the total motile sperm on top chamber divided the number of motile sperm in the raw stock semen sample). (C) The percentage of the PR sperm before and after sorting by a thermal gradient system or without temperature gradient system (Each point represents the measuring result from the one subject of the experiment).
Figs. 6 shows the sperm parameters of raw semen and sorting with or without temperature gradient. Comparison of the sperm parameters after sorting by the present invention with and without temperature gradient. VAP: average path velocity, VSL: straight-line velocity, VCL: curvilinear velocity.
Figs. 7 shows the sperm DNA fragmentation in processed fraction. Immunostaining of spermatozoa with TUNEL assay. Sperm sorting by the present invention reduced DNA fragmentation.
Figs. 8 are the schematic illustrations of the present invention for sorting functional human sperm. (A) top view, (B) cross-sectional view of upper chamber; (C) top view, (D) cross-sectional view of bottom chamber; (E) assembly device.
Figs. 9 shows sorting with the present invention with plurality of through-holes reduced immotile sperm after sorting. The upper chamber was designed with plurality of through-holes and examined sperm recovery after sorting.

### DETAILED DESCRIPTION OF THE INVENTION

All the features disclosed in this specification may be combined in any combination. An alternative feature serving the same, equivalent, or similar purpose may replace each feature disclosed in this specification. Thus, unless expressly stated otherwise, each feature disclosed is only an example of a generic series of equivalent or similar features.

### Device fabrication

As shown in Fig. 1, the device provided herein generally comprises a bottom chamber **(10)** and an upper chamber **(20)** for semen sample deposits and the collection of sorted spermatozoa, respectively. The bottom chamber **(10)** has an injection port **(11)** for sample injection, while the upper chamber has a collection port **(21)** for loading buffer and harvesting sorted spermatozoa. A temperature control unit **(400)** is utilized to maintain the temperature of the upper chamber **(20)** higher than that of the bottom chamber **(10).** A porous layer **(30)** with a typical pore diameter of 8 to 20 µm was placed between two main chambers which could help to maintain the temperature gradient as well as allow motile sperm to travel from the bottom chamber **(10)** to the upper chamber **(20).**

Typically, the temperature difference between the upper chamber **(20)** and the bottom chamber **(10)** is maintained to be 1-4 °C, for example, the temperatures of the upper chamber **(20)** and bottom chamber **(10)** were kept at 35-38 °C and 30-36 °C, respectively. During the operation, the motile sperm **(50)** gradually accumulate in the upper chamber **(20),** while the immotile sperm **(60)** stay in the bottom chamber **(10).** Examples of the temperature control unit **(400)** can be a heating pad **(410),** a thermal gradient incubator **(420),** or simply a pre-warmed metal sheet.

A heating source such as a heating pad **(410)** or a pre-warmed metal sheet warms the fluid inside by contacting the outer walls of the upper chamber. The heating pad **(410)** is set to maintain a temperature. The pre-warmed metal sheet pre-warmed to a temperature before operation. Preferably, the said temperatures are slightly higher than the desired temperature of the fluid in the upper chamber.

A thermal gradient incubator **(420)** is designed to provide the heat from the one end of the heating source **(421).** The other side is either subjected to free cooling by providing a plurality of holes **(422)** of the thermal gradient incubator.

The porous layer **(30)** separating the upper and lower chambers not only concentrates the spermatozoa swum-up against gravity **(50)** in the upper chamber (20) and leaves immotile spermatozoa **(60)** in the bottom chamber **(10),** but also delays the thermal equilibrium of the upper and bottom chambers. Typically, the porous layer **(30)** is a biocompatible membrane, either hydrophobic or hydrophilic. In some embodiments, the biocompatible membrane is a polycarbonate track-etched membrane or a polyvinyl alcohol (PVA) membrane.

### Human sperm handling and sperm sorting

Human semen samples were obtained from donors after 3 days of sexual abstinence. Informed consent was obtained from each donor. After obtaining semen samples from the hospital, liquefied-semen samples were then analyzed by LensHooke X1 pro (bonraybio). Subsequently, a semen sample was split into two fractions for sperm sorting in the presence and absence of temperature gradient condition. Spermatozoa were separated from crude semen by the present invention. Briefly, a semen sample of 1.5~2 mL was injected into the bottom of the device; the upper part of the device was filled with 1~1.5 mL of sperm washing medium (mHTF medium). Recovered sperms were collected after 15 to 30 minutes incubation in the presence or absence of heating pad. Forty microliters of recovery samples were placed on a LensHooke CS0 chip (bonraybio) and analyzed with LensHooke X1 pro (bonraybio).

### Immunofluorescence staining

To compare the quality of spermatozoa before and after sorting, approximately 2×10⁶ spermatozoa were centrifuged at 400 g for 7 minutes and resuspended in PBS containing 4% of paraformaldehyde. After fixation, the samples were centrifuged and washed with PBS twice, and then resuspended in PBS at 200 µL. Fixed cells were then aliquoted and smeared onto a glass microscope slide and left to dry. The air-dried slides can be placed into storage at -80 °C or performed immunofluorescence staining directly. The assessment of sperm DNA fragmentation (sDF) in spermatozoa was evaluated by using with TUNEL assay.

### Example 1: Vertical thermal gradient establishment

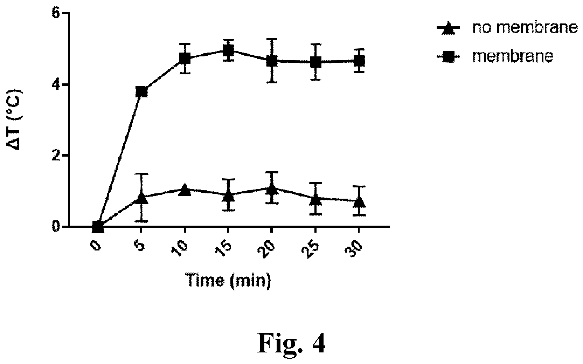

In this and the following examples, the temperature control unit (400) comprises a heating source **(410)** in thermal contact with the outer wall of the upper chamber **(20)** to generate a temperature gradient between the upper and bottom chambers, and the porous layer **(30)** is a polycarbonate track-etched membrane filter. A vertical thermal gradient system was set up as described in Fig. 2, the temperature of the mild heat source was detected by an Infrared thermal imager (MT-4606, Proskit). Inserting the porous PCTE membrane not only allows motile sperm to pass through from the bottom chamber to the upper chamber but also has the direct benefit of enhancing the temperature gradient as illustrated in Fig. 4. The result showed that the average temperature difference between the upper and bottom chamber in the presence and absence of membrane were determined at the range of 3.80~4.97 °C and 0.73~1.10 °C in 30 minutes, respectively. Temperature gradient was effective in the whole measured range. The results suggest that placing the porous layer **(30)** between the sorting device can maintain a temperature gradient of at least 3 °C. Compared with the prior horizontal temperature-gradient system for spermatozoa isolation, the temperature gradient can be achieved in a space of only 4 mm (4 mm for the upper chamber, 4 mm for the bottom chamber), and only a simple heating source is required.

### Example 2 Sperm motility, concentration, and isolation efficiency

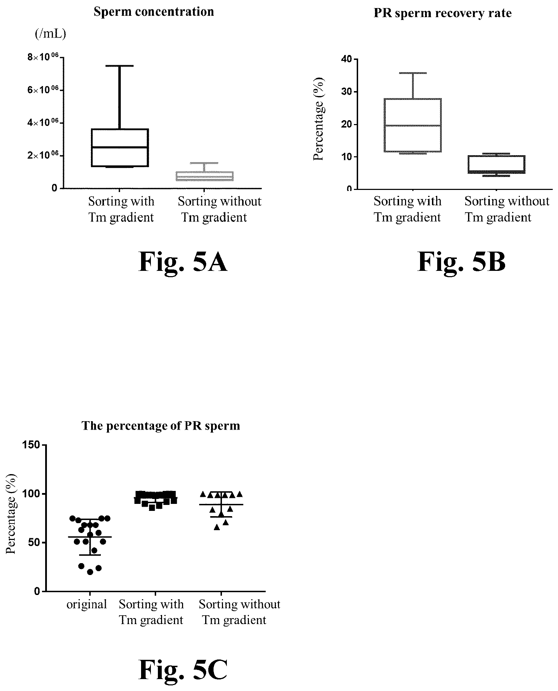

The improvement of sperm sorting with a vertical thermal gradient system was assessed in parallel to sperm sorting without a vertical thermal gradient system. Once the sperm was collected from the upper chamber **(20),** we analyzed the concentration, motility as well as sperm parameters by CASA (LensHooke X1 pro bonraybio). As shown in Figure 5, sperm sorting utilizing a vertical thermal gradient exhibited an increase in total sperm counts compared to sperm sorting at a uniform temperature, with an increase of approximately 1.86- to 6-fold (Figure 5A).

In Figure 5B, the recovery rate of progressive sperm (PR sperm) was increased approximately two- to four-fold in the sperm sorting with a vertical thermal gradient as compared with control (sorting with uniform temperature). Importantly, these results also demonstrated that the vertical thermal gradient system provieds a higher percentage of PR sperm (96 ± 4.86 %) as compared with the uniform temperature system (89.27 ± 12.71 %) and unsorted sample (54 ± 16.84 %).

### Example 3 Sperm velocity analysis

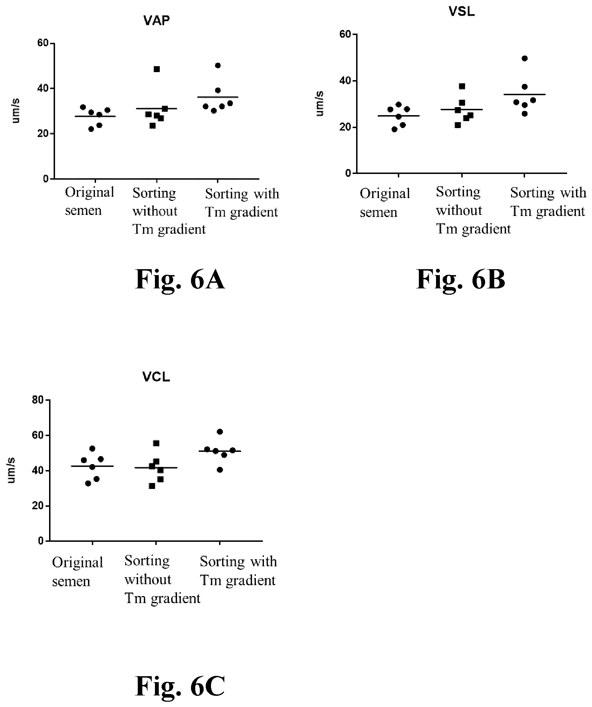

The sperms and moving paths of the sorted sperm were tracked by the computer-aided sperm analysis (LensHooke X1 pro bonraybio), as shown in Figs. 6 (A-C). The thermotaxis-sorted sperm showed a series of velocity parameters significantly higher than that of the raw sperm sample. The values of VAP, VSL, and VCL tend to increase in the vertical thermal gradient system, suggesting that sperm that responded to temperature gradient possess better velocity parameters.

### Example 4 Quality of thermotactic spermatozoa

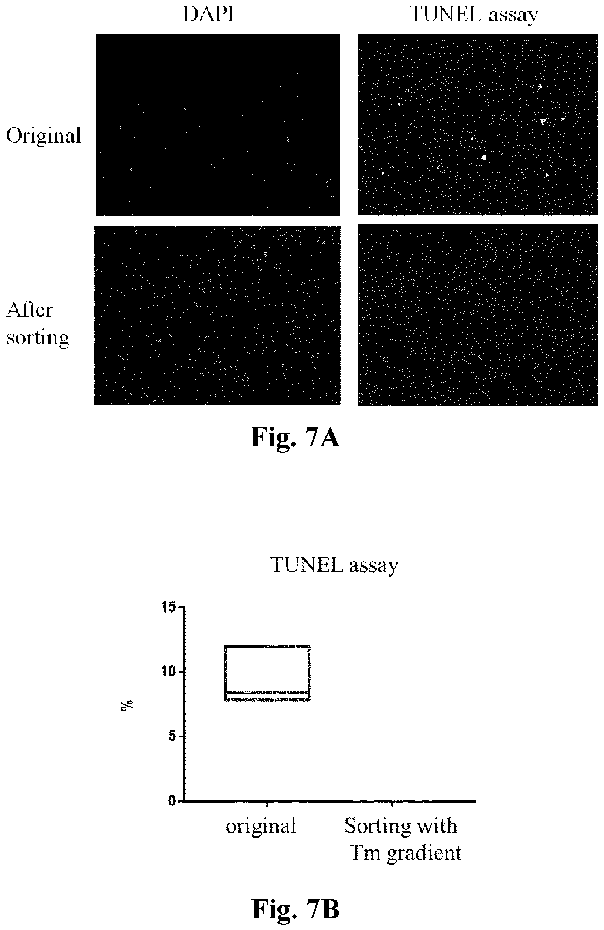

To assess the genetic quality of spermatozoa selected by thermotaxis, we examined DNA fragmentation level after sorting with our vertical sorting system by TUNEL assay. As shown in Figs. 7, the TUNEL index in spermatozoa after being selected by a vertical thermal gradient system was reduced to almost 0 % as compared to the unselected sample.

### Example 5 The upper chamber with plurality of through-holes reduced immotile cell number after sorting

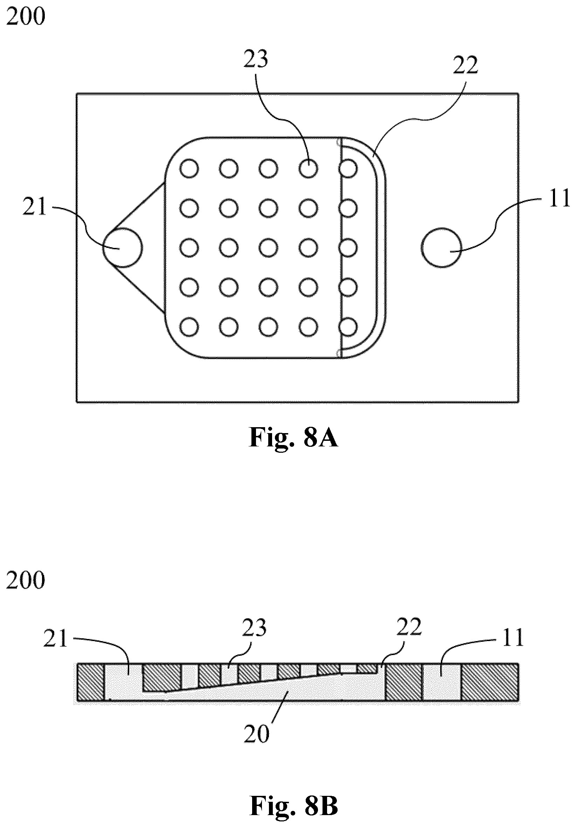

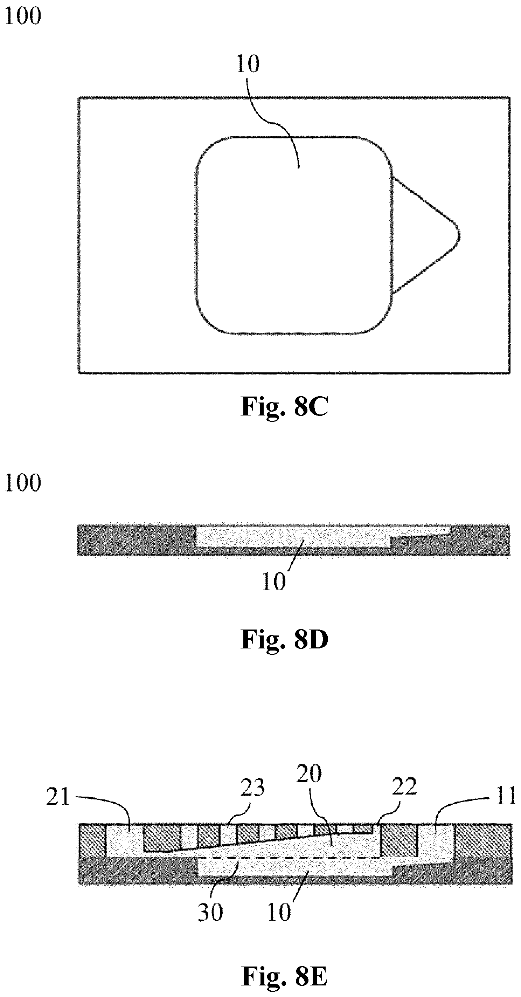

Bubbles were frequently observed when injecting buffer into the upper chamber (20). Sloping the ceiling of the upper chamber (20) and deploying a strip vent **(22)** at the higher end of the slope was found helpful to improve the situation. The bubbles arise during buffer injection contact the ceiling, move along the slope, and finally release through the strip vent **(22).** On the other hand, further disposing a plurality of through-holes **(23)** on the ceiling can relieve the negative pressure generated in the upper chamber **(20)** when collecting the sorted sperm through the collection port **(21).** Figs. 8A and 8B exemplify one of embodiments to achieve the above objectives.

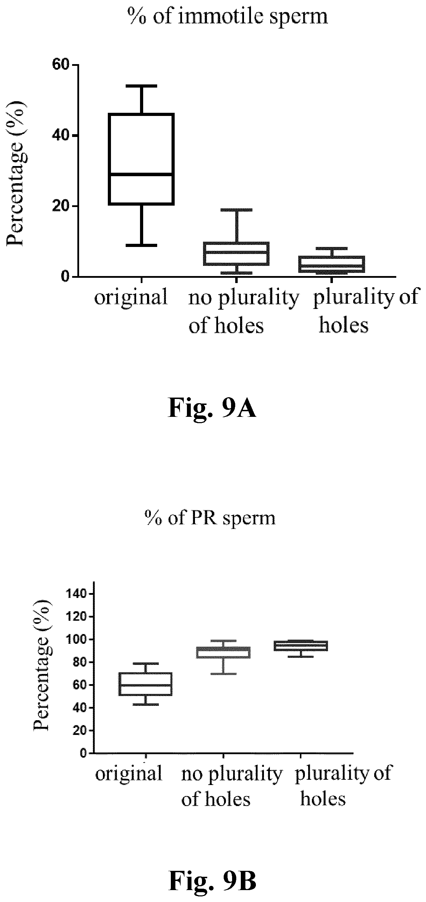

The performance of the devices with or without through-holes **(23)** in sperm sorting was compared at a uniform temperature (37 °C), as shown in Figs. 9. the sorting device with through-holes **(23)** reduced immotile cell number after sorting. The result indicates that increasing number of through-holes **(23)**on the ceiling is effective ways to reduce the number of immotile sperm when performing sperm sorting.

This invention imitates the avigation mechanism of spermatozoa in the female genital tract, it could be used as a tool in the ART setting to select the best spermatozoa as human spermatozoa after capacitation responds positively to a temperature gradient change.

## Claims

1. An apparatus for processing sperm, comprising:
an upper chamber and a bottom chamber arranged vertically;
a collection port connected to the upper chamber;
an injection port connected to the bottom chamber;
a porous layer placed at the interface between the upper and bottom chamber to allow particles communication between them and delay thermal equilibrium therebetween; and
a temperature control unit means for maintaining the temperature of the upper chamber higher than that of the bottom chamber,
wherein the temperature control unit comprises a heating source placed on top of the upper chamber to heat the upper chamber by contacting.

2. The apparatus of claim 1, wherein the porous layer is a biocompatible semipermeable membrane.

3. The apparatus of claim 2, wherein the biocompatible semipermeable membrane is a polycarbonate or polyvinyl alcohol track-etched membrane.

4. The apparatus of claim 1, wherein the pore diameter of the porous layer is between 8 to 20 µm.

5. The apparatus of claim 1, wherein the upper chamber comprises an inclined ceiling, the lower end of the inclined ceiling is close to the collection port and the higher end of inclined ceiling is away from the collection port.

6. The apparatus of claim 5, wherein the inclined ceiling comprises a strip vent at the higher end to release air bubbles.

7. The apparatus of claim 1, wherein the inclined ceiling comprises a plurality of through-holes to ventilate the upper chamber.

8. A method for processing sperm using the apparatus of claim 1, comprising:
a) providing an apparatus of claim 1;
b) injecting a population of unsorted sperm into the bottom chamber through the injection port;
c) injecting a compatible buffer into the upper chamber through the collection port;
d) maintaining the temperature of the upper chamber higher than that of the bottom chamber;
e) incubating a period;
f) harvesting a population of sorted sperm from upper chamber through the collection port.

9. The method of claim 8, wherein the temperature of the upper chamber is higher than that of the bottom by 1 to 4 °C.

10. The method of claim 9, wherein the temperatures of the upper and bottom chambers are maintained between 35 to 38 °C and 30 to 36 °C, respectively.

11. The method of claim 8, wherein the period is between 15 to 30 minutes.

12. The method of claim 8, wherein the compatible buffer is a bicarbonate or HEPES buffered sperm washing medium.

## Patentansprüche

1. Vorrichtung zum Verarbeiten von Sperma, die aufweist:
eine obere Kammer und eine untere Kammer, die vertikal angeordnet sind;
eine Sammelöffnung, die mit der oberen Kammer verbunden ist;
eine Injektionsöffnung, die mit der unteren Kammer verbunden ist;
eine poröse Schicht, die an der Grenzfläche zwischen der oberen und unteren Kammer angeordnet ist, um Teilchenkommunikation zwischen ihnen zu ermöglichen und thermisches Gleichgewicht zwischen ihnen zu verzögern; und
eine Temperatur-Steuer--Einheit zum Halten der Temperatur der oberen Kammer höher als die der unteren Kammer,
wobei die Temperatur-Steuer-Einheit eine Heizquelle aufweist, die oben auf der oberen Kammer platziert ist, um die obere Kammer mittels Kontaktierens zu erwärmen.

2. Vorrichtung nach Anspruch 1, wobei die poröse Schicht eine biokompatible semipermeable Membran ist.

3. Vorrichtung nach Anspruch 2, wobei die biokompatible semipermeable Membran eine spurgeätzte Membran aus Polycarbonat oder Polyvinylalkohol ist.

4. Vorrichtung nach Anspruch 1, wobei der Porendurchmesser der porösen Schicht zwischen 8 bis 20 µm ist.

5. Vorrichtung nach Anspruch 1, wobei die obere Kammer eine geneigte Decke aufweist, das untere Ende der geneigten Decke nahe der Sammelöffnung ist und das höhere Ende der geneigten Decke von der Sammelöffnung entfernt ist.

6. Vorrichtung nach Anspruch 5, wobei die geneigte Decke am oberen Ende einen Entlüftungsstreifen aufweist, um Luftblasen abzulassen.

7. Vorrichtung nach Anspruch 1, wobei die geneigte Decke mehrere Durchgangslöcher aufweist, um die obere Kammer zu belüften.

8. Verfahren zum Verarbeiten von Sperma unter Verwendung der Vorrichtung nach Anspruch 1, das aufweist:
a) Bereitstellen einer Vorrichtung nach Anspruch 1;
b) Injizieren einer Population von unsortiertem Sperma in die untere Kammer hinein durch die Injektionsöffnung;
c) Injizieren eines kompatiblen Puffers in die obere Kammer hinein durch die Sammelöffnung;
d) Halten der Temperatur der oberen Kammer höher als die der unteren Kammer;
e) Inkubieren für einen Zeitraum;
f) Ernten einer Population sortiertes Sperma aus der oberen Kammer durch die Sammelöffnung.

9. Verfahren nach Anspruch 8, wobei die Temperatur der oberen Kammer um 1 bis 4 °C höher ist als die der unteren.

10. Verfahren nach Anspruch 9, wobei die Temperaturen der oberen und unteren Kammern entsprechend zugeordnet zwischen 35 bis 38 °C bzw. 30 bis 36°C gehalten werden.

11. Verfahren nach Anspruch 8, wobei der Zeitraum zwischen 15 bis 30 Minuten ist.

12. Verfahren nach Anspruch 8, wobei der kompatible Puffer ein Bicarbonat- oder HEPES-gepuffertes Sperma-Wasch-Medium ist.

## Revendications

1. Appareil pour le traitement de sperme, comprenant :
une chambre supérieure et une chambre inférieure disposées verticalement ;
un orifice de collecte relié à la chambre supérieure ;
un orifice d'injection relié à la chambre inférieure ;
une couche poreuse placée à l'interface entre la chambre supérieure et la chambre inférieure pour permettre la communication de particules entre celles-ci et retarder l'équilibre thermique entre celles-ci ; et
un moyen d'unité de contrôle de température pour maintenir la température de la chambre supérieure supérieure à celle de la chambre inférieure,
dans lequel l'unité de contrôle de température comprend une source de chauffage placée au-dessus de la chambre supérieure pour chauffer la chambre supérieure par contact.

2. Appareil selon la revendication 1, dans lequel la couche poreuse est une membrane semi-perméable biocompatible.

3. Appareil selon la revendication 2, dans lequel la membrane semi-perméable biocompatible est une membrane de polycarbonate ou d'alcool polyvinylique gravée par rayons.

4. Appareil selon la revendication 1, dans lequel le diamètre de pores de la couche poreuse est compris entre 8 à 20 µm.

5. Appareil selon la revendication 1, dans lequel la chambre supérieure comprend un plafond incliné, l'extrémité inférieure du plafond incliné est proche de l'orifice de collecte et l'extrémité supérieure du plafond incliné est éloignée de l'orifice de collecte.

6. Appareil selon la revendication 5, dans lequel le plafond incliné comprend une bande d'aération à l'extrémité supérieure pour libérer les bulles d'air.

7. Appareil selon la revendication 1, dans lequel le plafond incliné comprend une pluralité de trous traversants pour ventiler la chambre supérieure.

8. Procédé de traitement de sperme en utilisant l'appareil selon la revendication 1, comprenant :
a) la fourniture d'un appareil selon la revendication 1 ;
b) l'injection d'une population de spermatozoïdes non triés dans la chambre inférieure à travers l'orifice d'injection ;
c) l'injection d'un tampon compatible dans la chambre supérieure à travers l'orifice de collecte ;
d) le maintien de la température de la chambre supérieure plus élevée que celle de la chambre inférieure ;
e) l'incubation pendant une période ;
f) la récolte d'une population de spermatozoïdes triés à partir de la chambre supérieure à travers l'orifice de collecte.

9. Procédé selon la revendication 8, dans lequel la température de la chambre supérieure est supérieure à celle de la chambre inférieure de 1 à 4 °C.

10. Procédé selon la revendication 9, dans lequel les températures des chambres supérieure et inférieure sont maintenues respectivement entre 35 à 38 °C et 30 à 36 °C.

11. Procédé selon la revendication 8, dans lequel la période est comprise entre 15 et 30 minutes.

12. Procédé selon la revendication 8, dans lequel le tampon compatible est un milieu de lavage de sperme tamponné au bicarbonate ou à l'HEPES.
